(19) Europäisches Patentamt
European Patent Office
Office européen des brevets

(11) Publication number: 0 324 626 A1

(12) **EUROPEAN PATENT APPLICATION**

(21) Application number: 89300280.8

(22) Date of filing: 12.01.89

(51) Int. Cl.4: C 07 D 211/90
A 61 K 31/445

(30) Priority: 13.01.88 KR 17888 13.01.88 KR 17988

(43) Date of publication of application: 19.07.89 Bulletin 89/29

(84) Designated Contracting States: DE ES FR GB IT

(71) Applicant: KOREA RESEARCH INSTITUTE OF CHEMICAL TECHNOLOGY
100, Chang-dong Jung-ku
Daejeon-si Chungcheongnam-do (KR)

(72) Inventor: Kim, Wan Joo Asia Seonsoochon Apt. 11-404
86 Chamsil-dong Kangdong-ku
Seoul (KR)

Shim, Young Key Gongdonggwanri Apt. 8-503
431, Doryong-dong Chung-ku
Daejeon-si Chungcheongnam-do (KR)

Chun, Jae Sang
10/4, 135-91, Daesa-dong Chung-ku
Daejeon-si Chungcheongnam-do (KR)

(74) Representative: Holdcroft, James Gerald, Dr. et al
Graham Watt & Co. Riverhead
Sevenoaks Kent TN13 2BN (GB)

# (54) 1,4-Dihydropyridine derivatives.

(57) 1,4-dihydropyridine derivatives represented by the formula:

R3
O
O
(I)
R1O
OR2
H3C
N
H
CH3

wherein
$R^1$ and $R^2$, different from the other, is selected from the group consisting of lower alkyl, lower alkenyl, lower alkoxyalkyl or aminoaryl(lower)alkyl having carbon atoms of 1 to 6, which may be substituted or unsubstituted by a lower alkyl having carbon atoms of 1 to 4, wherein at least one of $R^1$ and $R^2$ is a lower alkenyl having carbon atoms of 3 to 6 whose 1- or 2- position is substituted by a lower alkyl having carbon atoms of 1 to 4; and $R^3$ is a nitro group in ortho-, meta- or para-position of the phenyl.

The compounds are useful as vasodilators and in the treatment of hypertension.

EP 0 324 626 A1

**Description**

## 1,4-DIHYDROPYRIDINE DERIVATIVES

Background of the Invention

The present invention relates to novel 1,4-dihydropyridine derivatives having excellent effects for cerebral vascular vasodilation and showing lower toxicities.

Numerous calcium channel antagonists have been developed since Nifedipine as calcium channel antagonist was invented by the Bayer Co. in Germany in 1968.

Recently, a variety of ester derivatives of 1,4-dihydropyridine-3,5-dicarboxylic acid have been known as calcium channel antagonists, and especially unsymmetrical esters thereof have been exposed to take far superior effect and their preparative methods have been steadily studied until now.

As a method of preparing unsymmetrical esters of 1,4-dihydropyridine-3,5-dicarboxylic acid, for example, a method that an aminocrotonate having a suitable ester group and a benzaldehyde are reacted under the condition of the Hantzsch reaction ; another method that a benzylidene prepared by previously reacting a benzaldehyde with an acetoacetate, is reacted with an aminocrotonate ; the other method that only one side ester group of symmetrical ester thereof is transformed by using metal alkoxides, and the like, have been widely known.

Then, among said unsymmetrical ester compounds of 1,4-dihydropyridine, there are also reported compounds comprising ester groups in 3- and 5-positions of saturated or unsaturated hydrocarbons having side chains. And recently, there are actively progressing studies for developing novel unsymmetrical esters of 1,4-dihydropyridine-3,5-dicarboxylic acid.

For example, in the JPK No. 74-109384, there is disclosed that 3-β-(N-benzyl-N-methylamino)ethyl 5-methyl 2,6-dimethyl-4-(3′-nitrophenyl)-1,4-dihydropyridine-3,5-dicarboxylate shown by the following formula (I′) possess an antihypertensive property.

(I')

As the result that the present inventors selectively investigated pharmaceutically active ones headed by the compound (I′) of various diester compounds of 1,4-dihydropyridine which have been reported up to date, we became to find that unsymmetrical ester compounds comprising lower alkyl ester groups without severely change from fundamental structure thereof, showed special effects as antihypertensive agents, nevertheless, such compounds had a disadvantage in toxicity.

Therefore, while having ceaselessly studied with consideration to the foregoing points, the present inventors have prepared and tested numerous unsymmetrical ester compounds which comprised at least one alkenylester group having a lower alkyl side chain and lower alkenyl ester derivatives whose 1- or 2- positions was substituted especially by a lower alkyl group such as methyl group. As a result, we obtained novel 1,4-dihydropyridine derivatives possessing excellent antihypertensive action whose effecting time was long and exhibiting lower toxicity to accomplish the present invention.

Summary of the Invention

An object of the present invention is to provide novel ester derivatives of 1,4-dihydropyridine which particularly have excellent effects for treatment of hypertension and show lower toxicities and which have been not reported and suggested until now.

Brief description of the Drawings

Figure 1 is a polygraph showing blood pressure and heart rate which were measured using SHR before and after administration of the test compound in order to examine the blood pressure-reducing effect of

the compound and the effect duration time thereof ;

Figures 2A to 2L are resultant graphs showing change rates(%) of blood pressure and heart rate, depending upon doses, which were measured before and after administration of the test compounds in the respectively ;

Figure 3 is a resultant graph of the test for vasodilation effects of the compounds prepared in accordance with the present invention, using guinea-pig's pulmonary artery ; and

Figure 4 is a resultant graph of the test for negative inotropic effects of the compounds prepared in accordance with the present invention.

Detailed Description of the Invention

The present invention relates to novel unsymmetrical ester derivatives of 1,4-dihydropyridine which are shown by the following formula

(I)

wherein

$R^1$ and $R^2$, different from the other, is selected from the group consisting of lower alkyl, lower alkenyl, lower alkoxyalkyl or aminoaryl(lower)alkyl having carbon atoms of 1 to 6, which may be substituted or unsubstituted by a lower alkyl having carbon atoms of 1 to 4, wherein at least one of $R^1$ and $R^2$ is a lower alkenyl having carbon atoms of 3 to 6 whose 1- or 2- position is substituted by a lower alkyl having carbon atoms of 1 to 4 ; and

$R^3$ is a nitro group in ortho-, meta- or para- position of the phenyl.

The compounds of the present invention may be prepared by reacting a benzaldehyde derivative of the formula (II) with a lower alkyl, lower alkenyl, lower alkoxyalkyl or aminoaryl(lower)alkyl aminocrotonate of the formula(III), and a lower alkyl, lower alkenyl, lower alkoxyalkyl or lower aminoaryl(lower)alkyl acetoacetate of the formula (IV), at a time or in the optional order.

Said process can be represented as follows :

wherein, $R^1$, $R^2$ and $R^3$ are the same as defined above.

The enamine derivatives of the formula (III) among said three reactants may be prepared by reacting an acetoacetate with an excess of primary amine, and the compounds of the formula (IV) may be prepared, for example, by the ester interchange using of a methyl acetoacetate and a various alcohols in equimolar, or by reaction of a diketene with alcohols.

According to the present invention, the derivatives of 1,4-dihydropyridine shown by the formula (I) are prepared by the reaction of three compounds of the formulae (II), (III) and (IV) simultaneously, or by the reaction of the optional two compounds among said three compounds first and subsequently reacting the rest compound therewith. The process of the present invention are preferably carried out in any organic solvent such as ethanol, isopropanol, dioxane, dimethylformamide, dimethylsulfoxide, acetonitrile, and the like.

The new compounds of the formula (I) prepared as previously stated may be separated and purified by extraction, column chromatography, recrystallization and the like, and after, the obtained compounds may be optionally converted into the physiologically acceptable acid salts for pharmaceutical use. Such salts include those of inorganic acids, for example, hydrochloric acid, sulfuric acid, phosphoric acid and the like, and those of organic acids, for example, acetic acid, fumaric acid, maleic acid, tartaric acid, and the like.

Also optical isomers which can exist, of the compounds shown by the formula (I), are considered to be within the scope of the present invention.

In the foregoing formulae (I) to (IV), the term "lower alkyl" corresponding to $R^1$ and $R^2$ denotes methyl, ethyl, propyl, isopropyl, n-butyl, isobutyl, t-butyl or pentyl, "lower alkenyl" denotes alkenyl having carbon atoms of 3 to 6, which may be substituted or unsubstituted by a lower alkyl having carbon atoms of 1 to 4, and "aminoaryl(lower) alkyl" preferably denotes aminobenzyl methyl.

Especially, 1,4-dihydropyridine derivatives according to the present invention are significant in view that $R^1$ and $R^2$ in the formula (I) are different from each other, because such unsymmetrical compounds have much superior effects. Moreover, the compounds of the formula (I) have the best effect in case that at least one of $R^1$ and $R^2$ is 2-propenyl whose 1- or 2-position is substituted by a lower alkyl having carbon atoms of 1 to 4 and whose terminal, that is 3-position, is not substituted by any group.

Representative compounds (I) of the present invention include :

3-β-(N-benzyl-N-methylamino)ethyl 5-(1-methyl-2-propenyl) 2,6-dimethyl-4-(3′-nitrophenyl)-1,4-dihydropyridine-3,5-dicarboxylate

3-(1-methyl-2-propenyl) 5-isopropyl 2,6-dimethyl-4-(3′-nitrophenyl)-1,4-dihydropyridine-3,5-dicarboxylate

3-(1-methyl-2-propenyl) 5-methyl 2,6-dimethyl-4-(3′-nitrophenyl)-1,4- dihydropyridine-3,5-dicarboxylate

3-(1-methyl-2-propenyl) 5-ethyl 2,6-dimethyl-4-(3′-nitrophenyl)-1,4-dihydropyridine-3,5-dicarboxylate

3-(1-methyl-2-propenyl) 5-(2-methoxyethyl) 2,6-dimethyl-4-(3′-nitrophenyl)-1,4-dihydropyridine-3,5-dicarboxylate

3-(1-methyl-2-propenyl) 5-allyl 2,6-dimethyl-4-(3′-nitrophenyl)-1,4-dihydropyridine-3,5-dicarboxylate

3-(1-methyl-2-propenyl) 5-methyl 2,6-dimethyl-4-(2′-nitrophenyl)-1,4-dihydropyridine-3,5-dicarboxylate

3-(1-methyl-2-propenyl) 5-ethyl 2,6-dimethyl-4-(2′-nitrophenyl)-1,4-dihydropyridine-3,5-dicarboxylate

3-β-(N-benzyl-N-methylamino)ethyl 5-(2-methyl-2-propenyl) 2,6-dimethyl-4-(3′-nitrophenyl)-1,4-dihydropyridine-3,5-dicarboxylate

3-(2-methyl-2-propenyl) 5-isopropyl 2,6-dimethyl-4-(3′-nitrophenyl)-1,4-dihydropyridine-3,5-dicarboxylate

3-(2-methyl-2-propenyl) 5-methyl 2,6-dimethyl-4-(3′-nitrophenyl)-1,4-dihydropyridine-3,5-dicarboxylate

3-(2-methyl-2-propenyl) 5-ethyl 2,6-dimethyl-4-(3′-nitrophenyl)-1,4-dihydropyridine-3,5-dicarboxylate

3-(2-methyl-2-propenyl) 5-(2-methoxyethyl) 2,6-dimethyl-4-(3′-nitrophenyl)-1,4-dihydropyridine-3,5-dicarboxylate

3-(2-methyl-2-propenyl) 5-allyl 2,6-dimethyl-4-(3′-nitrophenyl)-1,4- dihydropyridine-3,5-dicarboxylate

3-(2-methyl-2-propenyl) 5-methyl 2,6-dimethyl-4-(3′-nitrophenyl)-1,4-dihydropyridine-3,5-dicarboxylate

3-(2-methyl-2-propenyl) 5-ethyl 2,6-dimethyl-4-(3′-nitrophenyl)-1,4-dihydropyridine-3,5-dicarboxylate

This invention will be further described in detail with the following examples. It should be noted that the present invention is not limited to these examples.

Example 1 : 3-β-(N-benzyl-N-methylamino)ethyl 5-(1-methyl-2-propenyl) 2,6-dimethyl-4-(3′-nitrophenyl)-1,4-dihydropyridine-3,5-dicarboxylate

1.06g(7.022mmoles) of 3-nitrobenzaldehyde, 1.10g (7.02moles) of N-benzyl-N-methylaminoethyl 3-aminocrotonate and 1.10g(7.022mmoles) of 1-methyl-2-propenyl acetoacetate were dissolved in 7mℓ of isopropylalcohol and refluxed for 6 hours. The solvent was distilled off under reduced pressure and purified by column chromatography to give 2.2g of the above-indicated compound in a pure state.

Yield : 60.4%

m.p. : 66 ~ 69°C

$^1$H NMR($CDCl_3$) : δ = 1.15, 1.30(d, 3H, $-CH_3$),
2.20(s, 3H, $-N-CH_3$), 2.30(s, 6H, $-CH_3$),
2.60(t, 2H, $-CH_2-N$), 3.45(s, 2H, $PhCH_2-$),
4.15(t, 2H, $-OCH_2-$), 4.70 ~ 5.35(m, 1H, -OCH<),
5.05(d, 2H, $=CH_2$), 5.25(s, 1H, $-C_4-H$),
5.40~5.90(m,1H, =CH-), 6.40(b, 1H, >NH),
7.20(s, 5H, Ph-), 7.30 ~8.15(m, 4H, aromatic).

Example 2 : 3-(1-methyl-2-propenyl) 5-isopropyl 2,6-dimethyl-4-(3′-nitrophenyl)-1,4-dihydropyridine-3,5-dicarboxylate

1.06g(7.022mmoles) of 3-nitrobenzaldehyde, 1.10g(7.022mmoles) of N-benzyl-N-methylaminoethyl 3-aminocrotonate and 1.10g(7.022mmoles) of 1-methyl-2-propenyl acetoacetate were dissolved in 7ml of isopropylalcohol and refluxed for 6 hours. The solvent was distilled off under reduced pressure and purified by column chromatography to give 2.2g of the above-indicated compound in a pure state.

Yield : 76%

m.p. : 58 ~60°C

$^1$H NMR($CDCl_3$) : δ = 1.00 ~1.40(m, 9H, $-CH_3$), 2.35(s, 6H, $-CH_3$),
5.06(d, 2H, $=CH_2$), 5.30(s, 1H, $C_4$-H),
4.75~5.40(m, 2H, -OCH<), 5.45 ~6.10(m, 1H, =CH-),
6.35(b, 1H, >NH), 7.20 ~8.15(m, 4H, aromatic).

Example 3 : 3-(1-methyl-2-propenyl) 5-(2-methoxyethyl) 2,6-dimethyl-4-(3′-nitrophenyl)-1,4-dihydropyridine-3,5-dicarboxylate

1.06g(7.022 mmoles) of 3-nitrobenzaldehyde, 1.10g(7.022mmoles) of 1-methyl-2-propenyl acetoacetate and 1.12g(7.022mmoles) of 2-methoxyethyl 3-aminocrotonate were dissolved in 7ml of isopropylalcohol. Hereinafter the procedure was the same as in Example 2 to give 1.6g of the above-indicated compound in a pure state.

Yield : 53%

m.p. : 98~99°C

$^1$H NMR($CDCl_3$) : δ = 1.17, 1.33(d, 3H, $-CH_3$), 2.35(s, 6H, $-CH_3$),
3.35(s, 3H, $-OCH_3$), 3.53(t, 2H, $-CH_2-OCH_3$),
4.17(t, 2H, $-OCH_2-$), 5.05(d, 2H, $=CH_2$),
4.70~5.35(m, 1H, -OCH<), 5.27(s, 1H, $C_4$-H),

5.40~6.10(m, 1H, =CH-), 6.5(b, 1H, >NH), 7.20~8.15(m, 4H, aromatic).

Example 4 : 3-(1-methyl-2-propenyl) 5-ethyl 2,6-dimethyl-4-(3′-nitrophenyl)-1,4-dihydropyridine-3,5-dicarboxylate

1.06g(7.022mmoles) of 3-nitrobenzaldehyde, 1.10g(7.022mmoles) of 1-methyl-2-propenyl acetoacetate and 0.91g (7.022mmoles) of ethyl 3-aminocrotonate were dissolved in 7ml of isopropylalcohol. Hereinafter the procedure was the same as in Example 2 to give 2.0g of the above-indicated compound in a pure state.
Yield : 71%
m.p. : 96 ~98°C
$^1$H NMR($CDCl_3$) : δ = 1.17, 1.33(d, 3H, -$CH_3$), 1.20(t, 3H, -$CH_3$) 2.33(s, 6H, -$CH_3$), 4.07(q, 2H, -$OCH_2$-), 5.05(d, 2H, -$CH_2$-), 5.27(s, 1H, $C_4$-H), 4.75 ~5.35(m, 1H, -OCH<), 5.40~6.10(m, 1H, =CH-), 6.35(b, 1H, >NH), 7.20~8.20(m, 4H, aromatic).

Example 5 : 3-methyl 5-(1-methyl-2-propenyl) 2,6-dimethyl-4-(3′-nitrophenyl)-1,4-dihydropyridine-3,5-dicarboxylate

1.06g(7.022mmoles) of 3-nitrobenzaldehyde, 1.10g(7.022mmoles) of 1-methyl-2-propenyl acetoacetate and 0.81g (7.022mmoles) of methyl 3-aminocrotonate were dissolved in 7ml of isopropylalcohol. Hereinafter the procedure was the same as in Example 2 to give 2.0g of the above-indicated compound in a pure state.
Yield : 73.8%
m.p. : 98 ~99°C
$^1$H NMR($CDCl_3$) : δ = 1.17, 1.32(d, 3H, -$CH_3$), 2.35(s, 6H, -$CH_3$) 3.63(s, 3H, -$OCH_3$), 5.05(d, 2H, =$CH_2$), 4.80 ~5.30(m, 1H, -OCH<), 5.30(s, 1H, $C_4$-H), 5.30 ~5.90(m, 1H, =CH-), 6.30(b, 1H, >NH), 7.15~8.15(m, 4H, aromatic).

Example 6 : 3-(1-methyl-2-propenyl) 5-allyl 2,6-dimethyl-4-(3′-nitrophenyl)-1,4-dihydropyridine-3,5-dicarboxylate

1.06g (7.022 mmoles) of 3-nitrobenzaldehyde, 1.10g(7.022mmoles) of 1-methyl-2-propenyl acetoacetate and 0.99g (7.022mmoles) of allyl 3-aminocrotonate were dissolved in 7ml of isopropylalcohol. Hereinafter the procedure was the same as in Example 2 to give 2.2g of the above-indicated compound in a pure state.
Yield : 76.5%
m.p. : 73 ~75°C
$^1$H NMR($CDCl_3$) : δ = 1.17, 1.33(d, 3H, -$CH_3$), 2.35(s, 6H, -$CH_3$) 4.53(d, 2H, -$OCH_2$-), 4.95~5.25(m, 4H, $CH_2$=), 5.27(s, 1H, $C_4$-H), 4.70~5.50(m, 1H, -OCH<), 5.50~6.10(m, 2H, -CH=), 6.40(b, 1H, >NH), 7.20~8.15(m, 4H, aromatic).

Example 7 : 3-(1-methyl-2-propenyl) 5-(2-methyl-2-propenyl) 2,6-dimethyl-4-(2′-nitrophenyl)-1,4-dihydropyridine-3,5-dicarboxylate

1.06g(7.022mmoles) of 3-nitrobenzaldehyde, 1.10g(7.022mmoles) of 1-methyl-2-propenyl acetoacetate and 0.10g (7.022mmoles) of 2-methyl-1-propenyl aminocrotonate were dissolved in 7ml of isopropylalcohol. Hereinafter the procedure was the same as in Example 2 to give 2.1g of the above-indicated compound in oil phase.
Yield : 70.2%
$^1$H NMR($CDCl_3$) : δ = 1.07, 1.30(d, 3H, -$CH_3$), 1.60(s, 3H, -$CH_3$) 2.27, 2.32(s, each 3H< -$CH_3$), 4.45(s, 2H, -$OCH_2$-), 4.70, 4.73(s, 2H, $CH_2$=), 4.85~5.45(m, 2H, -OCH<, -CH=), 5.90(s, 1H, $C_4$-H), 6.13(b, 1H, >NH), 7.05~7.80(m, 4H, aromatic).

Example 8 : 3-β-(N-benzyl-N-methylamino)ethyl 5-(2-methyl-2-propenyl) 2,6-dimethyl-4-(3′-nitrophenyl)-1,4-dihydropyridine-3,5-dicarboxylate

2.45g(16.18mmoles) of 3-nitrobenzaldehyde, 4.05g (16.18mmoles) of N-benzyl-N-methylaminoethyl 3-aminocrotonate and 2.52g(16.18mmoles) of 1-methyl-2-propenyl acetoacetate were dissolved in 15ml of isopropylalcohol. Hereinafter the procedure was the same as in Example 1 to give 5.56g of the hydrochloric acid salt of the above-indicated compound.
Yield : 61.8%
m.p. : 96 ~110°C
$^1$H NMR($CDCl_3$) : δ = 1.63(s, 3H, -$CH_3$), 2.17(s, 3H, -N-$CH_3$), 2.30, 2.33(s, each 3H, -$CH_3$), 2.60(t, 2H, -$CH_2$-N),

3.43(s, 2H, $PhCH_2$),
4.10(t, 2H, $-OCH_2-$), 4.40(s, 2H, $-OCH_2-$),
4.67 ~ 4.83(m, 2H, $=CH_2$), 5.07(s, 1H, $-C_4-H$),
6.03(b, 1H, >NH), 7.13(s, 5H, Ph-),
7.30 ~ 8.20(m, 4H, aromatic).

Example 9 : 3-(2-methyl-2-propenyl) 5-isopropyl 2,6-dimethyl-4-(3′-nitrophenyl)-1,4-dihydropyridine-3,5-dicarboxylate

1.33g(8.775mmoles) of 3-nitrobenzaldehyde, 1.37g(8.775 mmoles) of 2-methyl-2-propenyl acetoacetate and 1.26g (8.775 mmoles) of isopropyl 3-aminocrotonate were dissolved in 7ml of isopropylalcohol. Hereinafter the procedure was the same as in Example 2 to give 2.29g of the above-indicated compound in a pure state.

Yield : 63%
m.p. : 124 ~ 125°C
$^1H$ NMR($CDCl_3$) : δ = 1.08, 1.21(d, each 3H, $-CH_3$),
1.60(s, 3H, $-CH_3$),
2.27, 2.30(s, each 3H, $-CH_3$),
4.27 ~ 4.40(m, 2H, $-OCH_2-$),
4.63 ~ 5.10(m, 4H, -OCH<, $-CH_2=$, $C_4-H$),
5.97(b, 1H, >NH), 7.03 ~ 8.10(m, 4H, aromatic).

Example 10 : 3-(2-methyl-2-propenyl) 5-(2-methoxyethyl) 2,6-dimethyl-4-(3′-nitrophenyl)-1,4-dihydropyridine-3,5-dicarboxylate

1.33g(8.775mmoles) of 3-nitrobenzaldehyde, 1.37g(8.775mmoles) of 2-methyl-2-propenyl acetoacetate and 1.40g (8.775mmoles) of 2-methoxy ethyl 3-aminocrotonate were dissolved in 7ml of isopropylalcohol. Hereinafter the procedure was the same as in Example 2 to give 2.98g of the above-indicated compound in a pure state.

Yield : 78.8%
m.p. : 89 ~ 90°C
$^1H$ NMR($CDCl_3$) : δ = 1.8(s, 3H, $-CH_3$), 2.3(s, 6H, $-CH_3$),
3.35(s, 3H, $-OCH_3$), 3.53(t, 2H, $-CH_2-OCH_3$),
4.17(t, 2H, $-OCH_2-$), 5.05(d, 2H, $=CH_2$),
4.70 ~ 5.35(m, 1H, -OCH<), 5.27(s, 1H, $C_4-H$),
5.40 ~ 6.10(m, 1H, =CH-), 6.5(b, 1H, >NH),
7.20 ~ 8.15(m, 4H, aromatic).

Example 11 : 3-(2-methyl-2-propenyl) 5-ethyl 2,6-dimethyl-4-(3′-nitrophenyl)-1,4-dihydropyridine-3,5-dicarboxylate

1.33g(8.775mmoles) of 3-nitrobenzaldehyde, 1.37g(8.775mmoles) of 1-methyl-2-propenyl acetoacetate and 1.13g (8.775mmoles) of ethyl 3-aminocrotonate were dissolved in 7ml of isopropylalcohol. Hereinafter the procedure was the same as in Example 2 to give 2.42g of the above-indicated compound in a pure state.

Yield : 69%
m.p. : 118 ~ 119°C
$^1H$ NMR($CDCl_3$) : δ = 1.17(t, 3H, $-CH_3$), 1.60(s, 3H, $-CH_3$),
2.27, 2.30(s, each 3H, $-CH_3$), 3.97(q, 2H, $-OCH_2-$),
4.33(s, 2H, $-OCH_2-$), 4.70(s, 2H, $=CH_2$),
5.00(s, 1H, $C_4-H$), 6.07(b, 1H, >NH),
6.97 ~ 8.00(m, 4H, aromatic).

Example 12 : 3-methyl 5-(2-methyl-2-propenyl) 2,6-dimethyl-4-(3′-nitrophenyl)-1,4-dihydropyridine-3,5-dicarboxylate

1.33g(8.775mmoles) of 3-nitrobenzaldehyde, 1.37g(8.775mmoles) of 2-methyl-2-propenyl acetoacetate and 1.01g (8.775mmoles) of methyl 3-aminocrotonate were dissolved in 7ml of isopropylalcohol. Hereinafter the procedure was the same as in Example 8 to give 1.83g of the above-indicated compound in a pure state.

Yield : 54%
m.p. : 122 ~ 123°C
$^1H$ NMR($CDCl_3$) : δ = 1.60(s, 3H, $-CH_3$),
2.30, 2.33(s, each 3H, $-CH_3$),
3.57(s, 3H, $-OCH_3$), 4.37(s, 2H, $-OCH_2-$),
4.73(s, 2H, $=CH_2$), 5.03(s, 1H, $C_4-H$),
6.07(b, 1H, >NH), 7.03 ~ 8.07(m, 4H, aromatic).

Example 13 : 3-(2-methyl-2-propenyl) 5-allyl 2,6-dimethyl-4-(3′-nitrophenyl)-1,4-dihydropyridine-3,5-dicarboxylate

1.33g(8.775mmoles) of 3-nitrobenzaldehyde, 1.37g(8,775mmoles) of 2-methyl-2-propenyl acetoacetate and 1.235g(8.775mmoles) of allyl 3-aminocrotonate were dissolved in 7ml of isopropylalcohol. Hereinafter the

procedure was the same as in Example 8 to give 2.92g of the above-indicated compound in a pure state.
Yield : 81%
m.p. : 87~88°C
$^1$H NMR($CDCl_3$) : δ = 1.63(s, 3H, $-CH_3$), 2.33(s, 6H, $-CH_3$), 4.40(s, 4H, $-OCH_2-$), 4.53(s, 2H, $CH_2=C<$), 4.73(s, 2H, $=CH_2$), 5.03~5.27(m, 2H, $C_4$-H, -CH=), 6.63(b, 1H, >NH), 7.07~8.07(m, 4H, aromatic).

To demonstrate the superior effect of the new compounds, some of them were tested on the following methods.

Test for antihypertensive effect

Pentobarbital sodium (50mg/kg) is intraperitoneally administrated to SHR(Spontaneously hypertensive rate), and the SHR is anesthetized by intravenous administration of an anesthetic in a ratio of 5mg/kg/hour using infusion pump. The cathether is inserted into the right carotid artery of such anesthetized SHR, and the test compound is administrated via the cathether. At this moment, arterial blood pressure and heart rate before and after administration of the test compound are continuously measured through the catheterized artery with a pressure transducer, and the results are recorded on a polygraph.

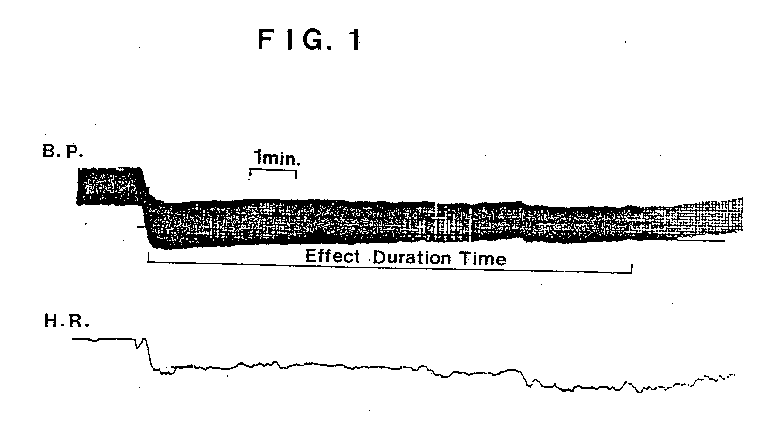

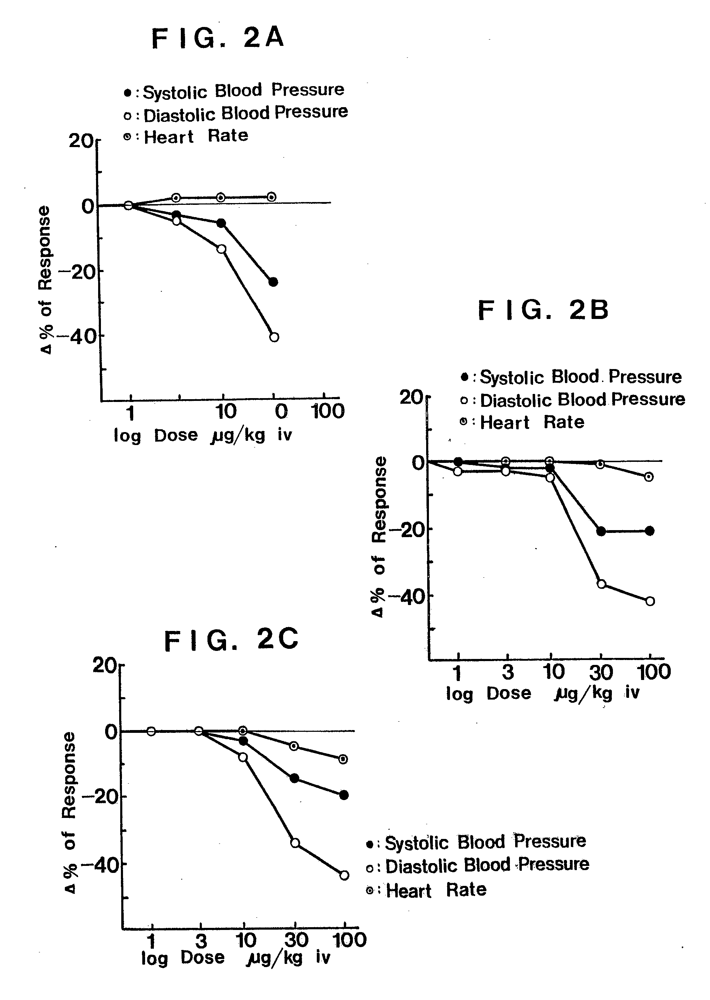

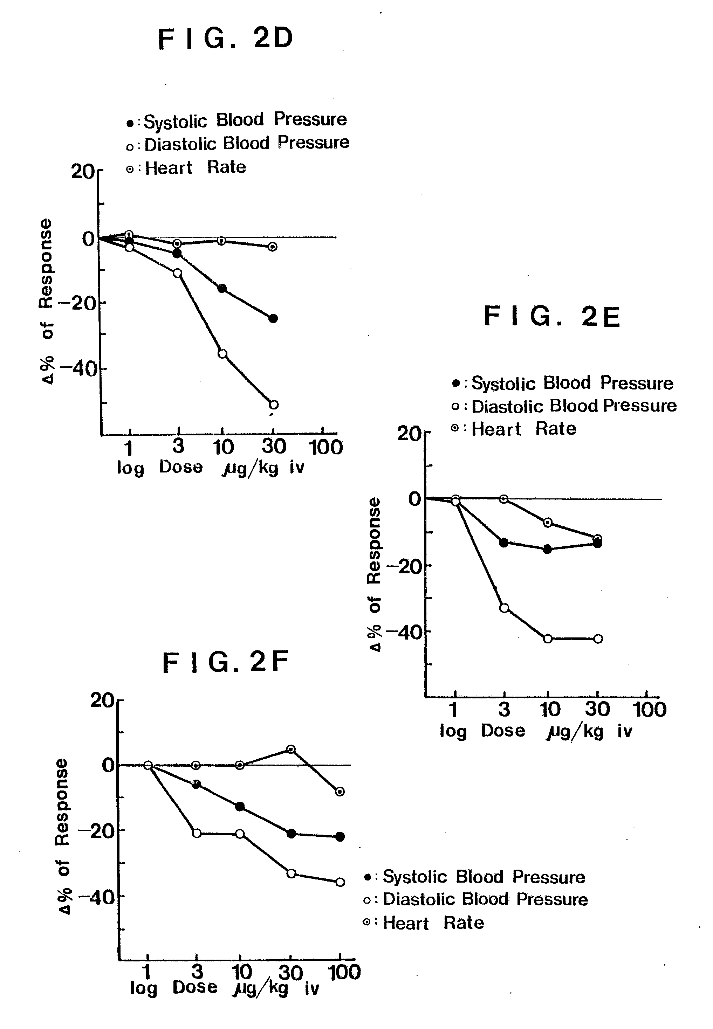

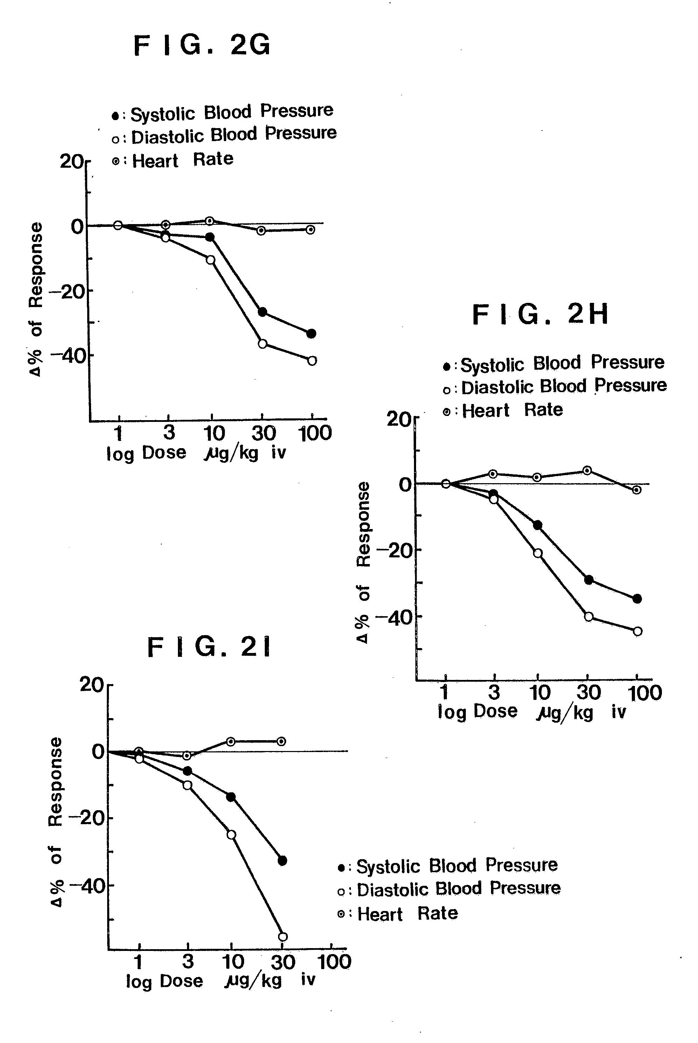

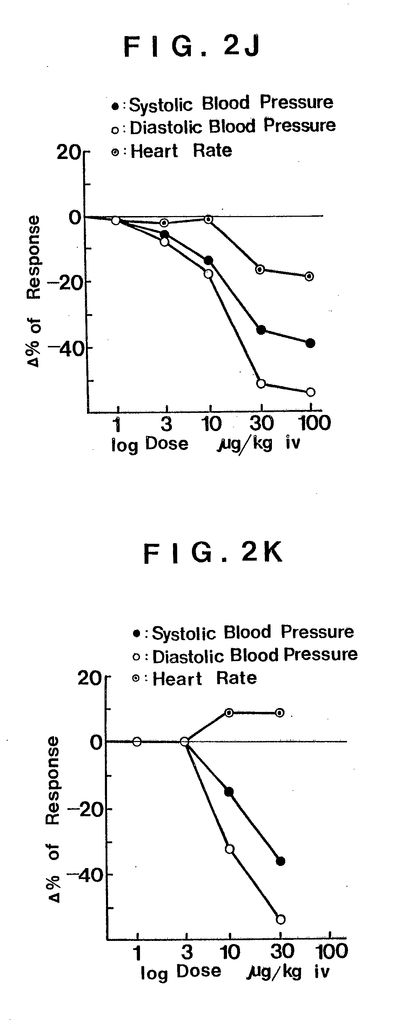

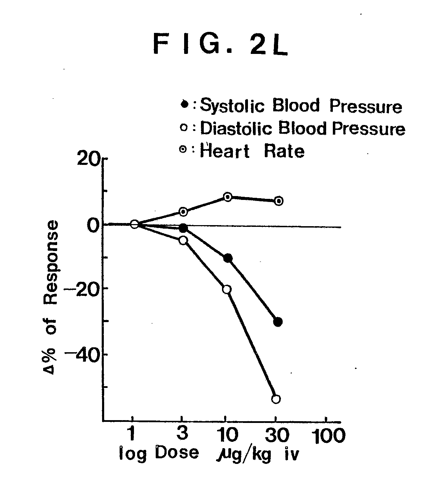

The blood pressure-reducing effect is represented by change rates(%) of blood pressure and heart rate which are measured before and after administration of the test compound with doses, and the effect duration time is determined on the basis of the time exhibiting 50% reduction of blood pressure against one exhibiting the maximum blood pressure reduction at 30 μg/kg bodyweight. The results are shown in Table 1, Figure 1 and Figures 2A to 2L.

## Table 1

| Test | Test Compound | The number of tested animals | Measured specifications | Dose, i.v. | | | | | Effect Duration time (min.) |
|---|---|---|---|---|---|---|---|---|---|
| | | | | 1μg/kg | 3μg/kg | 10μg/kg | 30μg/kg | 100μg/kg | |
| (1) | Example 4 | 3 | D.B.P. (1)<br>S.B.P. (2)<br>H.R. (3) | -<br>-<br>- | - 5± 2<br>- 3± 3<br>+ 2± 1 | -14± 4<br>- 6± 2<br>+ 2± 1 | -41± 4<br>-24± 4<br>+ 2± 1 | | 6.9 ± 2.5 |
| (2) | Example 2 | 3 | D.B.P.<br>S.B.P.<br>H.R. | - 3± 3<br>0<br>0 | - 3± 3<br>- 2± 2<br>0 | - 5± 1<br>- 2± 1<br>0 | -37± 9<br>-21± 2<br>- 1± 3 | - 42<br>- 21<br>- 5 | 4.8 ± 1.9 |
| (3) | Example 6 | 3 | D.B.P.<br>S.B.P.<br>H.R. | 0<br>0<br>0 | 0<br>0<br>0 | - 8± 2<br>- 3± 2<br>0 | -34± 2<br>-15± 1<br>- 5± 3 | -44± 5<br>-20± 6<br>- 9± 6 | 3.3 ± 1.1 |
| (4) | Example 3 | 3 | D.B.P.<br>S.B.P.<br>H.R. | - 3± 3<br>- 1± 1<br>+ 1± 1 | -11± 1<br>- 5± 2<br>- 2± 2 | -36± 3<br>-16± 1<br>- 1± 1 | -51± 8<br>-25± 2<br>- 3± 4 | | 8.3 ± 1.5 |
| (5) | Example 1 | 2 | D.B.P.<br>S.B.P.<br>H.R. | - 1± 1<br>- 1± 1<br>0 | -33±11<br>-13± 5<br>0± 1 | -42±12<br>-15± 4<br>- 7± 4 | -42<br>-13<br>-12 | | 10.7 |
| (6) | Example 12 | 3 | D.B.P.<br>S.B.P.<br>H.R. | 0<br>0<br>0 | -21± 8<br>- 6± 6<br>0 | -21±10<br>-13± 8<br>0± 1 | -33± 5<br>-21± 4<br>+ 5± 4 | -36± 3<br>-22± 4<br>- 8± 8 | 4.4±0.3 |

## Table 1(continued)

| Test | Test Compound | The number of tested animals | Measured specifications | Dose, i.v. | | | | | Effect Duration time (min.) |
|---|---|---|---|---|---|---|---|---|---|
| | | | | 1μg/kg | 3μg/kg | 10μg/kg | 30μg/kg | 100μg/kg | |
| (7) | Example 11 | 3 | D.B.P.<br>S.B.P.<br>H.R. | 0<br>0<br>0 | - 4± 4<br>- 3± 3<br>0 | -11± 6<br>- 4± 3<br>+ 1± 1 | -37± 4<br>-27± 5<br>- 2± 3 | -42± 3<br>-34± 3<br>- 2± 5 | 2.3± 0 |
| (8) | Example 9 | 2 | D.B.P.<br>S.B.P.<br>H.R. | 0<br>0<br>0 | - 5± 5<br>- 3± 3<br>+ 3± 2 | -21± 9<br>-13± 8<br>+ 2± 0 | -40± 8<br>-29± 5<br>+ 4± 3 | -45± 3<br>-35± 7<br>- 2± 5 | 3.3±0.6 |
| (9) | Example 13 | 4 | D.B.P.<br>S.B.P.<br>H.R. | - 2± 1<br>- 1± 1<br>0 | -10± 1<br>- 6± 2<br>- 1± 1 | -25± 4<br>-14± 6<br>+ 3± 2 | -56± 4<br>-33± 6<br>+ 3± 4 | | 6.0±0.3 |
| (10) | Example 10 | 3 | D.B.P.<br>S.B.P.<br>H.R. | - 1± 1<br>- 1± 1<br>- 1± 1 | - 8± 3<br>- 6± 3<br>+ 2± 2 | -18± 3<br>-14± 3<br>- 1± 2 | -51±10<br>-35± 8<br>-17±12 | -54± 3<br>-39± 8<br>-19±12 | 6.4±1.7 |
| (11) | Example 8 | 2 | D.B.P.<br>S.B.P.<br>H.R. | 0<br>0<br>0 | -<br>-<br>- | -32± 4<br>-15± 1<br>+ 9± 2 | -54± 9<br>-36± 3<br>+ 9± 2 | | 10.2±2.2 |
| (12) | Nicardipine | 2 | D.B.P.<br>S.B.P.<br>H.R. | -<br>-<br>- | - 5± 1<br>- 1± 0<br>+ 4± 0 | -20± 1<br>-10± 1<br>+ 8± 1 | -53± 3<br>-30± 7<br>+ 7± 1 | | 3.6±0.8 |

〈Note〉 (1) : D.B.P. = Diastolic Blood Pressure
(2) : S.B.P. = Systolic Blood Pressure
(3) : H.R. = Heart Rate

Toxicity Test

To examine acute toxicities of the novel 1,4-dihydropyridine derivatives, some compounds which were prepared in the foregoing examples were orally administrated to SPF mice. The limit test and the range finding test were carried out using the SPF mice, as stated below, and $LD_{50}$ values were determined. At this time, Nifedipine and Nicardipine were tested for comparison. The results were shown in Table 2.

1. Limit test

The limit test is carried out using 5 male and 5 female SPF mice (ICR) having bodyweights of 20 to 30g(mean ± 20%) which have been adapted for 5 days, wherein the positive control group is used to examine the toxicity of vehicle itself. After the test compound is orally administrated into the stomach of the mice which have been fasted for 4 hours prior to administration, the clinical symptoms are observed 4 times in the first day and once a day to the 14th day, respectively. The dead mice are weighed and organ examinations are carried out for macroscopical lesions. Also, on the last day of the test all surviving mice are sacrificed and used for examinations.

2. Range finding test

If the mortality is more than 50% in the limit test, the range finding test is carried out for 14 days with three doses groups including the putative $LD_{50}$ value. The method is same as the foregoing limit test.

TABLE 2

| Test Compound | Toxicity<br>$LD_{50}$ mg/kg, p.o. |
|---|---|
| Example 1 | > 1,000 |
| Example 3 | > 5,000 |
| Example 8 | > 1,000 |
| Example 10 | > 5,000 |
| Example 12 | 1,000 |
| Example 13 | > 5,000 |
| Nifedipine | < 500 |
| Nicardipine | < 700 |

Test for pulmonary artery vasodilation

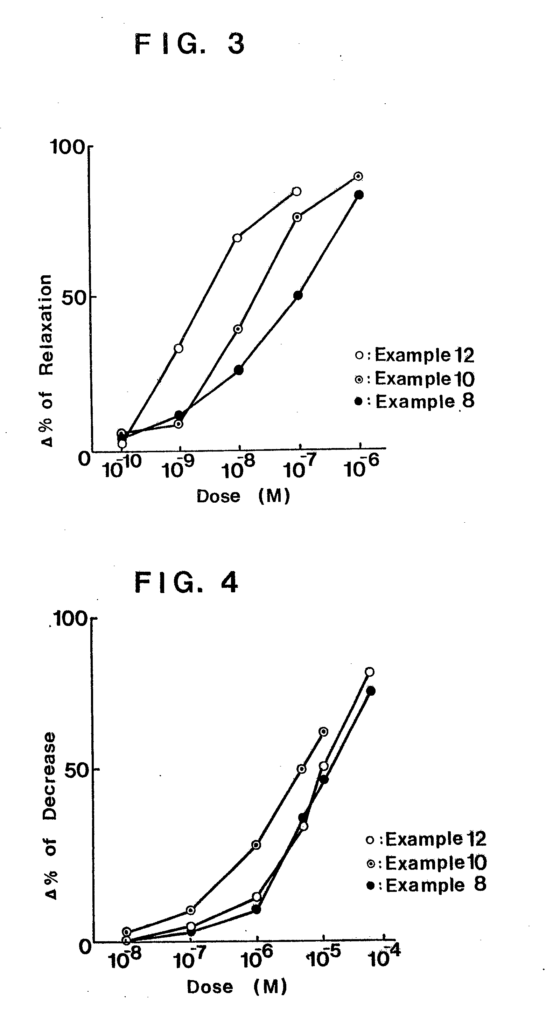

After a male guinea-pig was firstly fainted and exsanguinated, its pulmonary artery was cut in a length of 3~5mm and cut again into a spiral strip of 10~20mm in length in the Tyrode solution. The obtained strip was hung in the organ bath and sequently incubated with solution A, B and C which have been composed as in Table 3. Then, it was cumulatively applied with the test compound whose concentration was changed in a range of from $10^{-10}$ to $10^{-5}$M to obtain the dose-relaxation curve as shown in Figure 3. Also, the $EC_{50}$ values (i.e. the concentrations (M) of the test compound giving 50% of the relaxation rate) of the test compounds were listed in the following Table 4.

At this time, the relaxation rate was calculated on the basis of the following formula :

$$\text{Relaxation rate (\%)} = \frac{\text{degree of relaxation}}{\text{degree of maximum contraction}} \times 100$$

## Table 3

| | KCl | $CaCl_2$ | $MgSO_4$ | $KH_2PO_4$ | NaCl | $NaHCO_3$ | Glucose |
|---|---|---|---|---|---|---|---|
| <u>Sol.A (normal Tyrode)</u> ; 1hr. incubation (equilibration) | 0.37 | 0.13 | 0.14 | 0.14 | 6.55 | 2.10 | 2.27 |
| <u>Sol.B (Ca-free)</u> ; 5mins. incubation with EDTA (0.2mM), three times | 0.37 | - | 0.14 | 0.14 | 6.55 | 2.10 | 2.27 |
| <u>Sol.C (K-rich)</u> ; 1hr. incubation with $CaCl_2$ (0.5mM) (Ca-induced contraction) | 2.98 | - | 0.14 | 0.14 | 4.20 | 2.10 | 2.27 |

(Unit : g/ℓ)

Table 4

| | Test Compound | | |
|---|---|---|---|
| | Example 12 | Example 10 | Example 8 |
| $EC_{50}$ (M) | $2.1 \times 10^{-9}$ | $3.7 \times 10^{-8}$ | $1.0 \times 10^{-7}$ |

Test for negative inotropic effect

To examine the negative inotropic effect of the 1,4-dihydropyridine derivatives according to the present invention, the changes of contractility in the isolated left atrium of guinea-pig were monitored in a tissue chamber.

To elaborate on the procedure, after a male guinea-pig was exsanguinated, its left atrium was separated from the heart, and both ends thereof were fixed with holders in the tissue chamber. It was then stimulated by electric stimulator, wherein the voltage was about 10V which was higher as much as 10% than the threshold, and the duration time thereof was about 5MS. After maintaining about an hour until the contraction force became uniform, each test compound which has been dissolved in DMSO was cumulatively applied into the tissue chamber changing the concentration of the test compound from $10^{-8}$ to $10^{-4}$M.

The changes of contractility for isometric tension were monitored and their change percentages were calculated to obtain the dose-contraction decrease curve as shown in Figure 4.

As the results of the foregoing tests, it may be demonstrated that the compounds of this invention not only exhibit far superior blood pressure-reducing action with vasodilation effect whose effecting time is long but also have lower toxicities than prior compounds such as Nifedipine and Nicardipine.

**Claims**

1. 1,4-dihydropyridine derivatives represented by the formula

(I)

wherein

$R^1$ and $R^2$, different from the other, is selected from the group consisting of lower alkyl, lower alkenyl, lower alkoxyalkyl or aminoaryl(lower)alkyl having carbon atoms of 1 to 6, which may be substituted or unsubstituted by a lower alkyl having carbon atoms of 1 to 4, wherein at least one of $R^1$ and $R^2$ is a lower alkenyl having carbon atoms of 3 to 6 whose 1- or 2- position is substituted by a lower alkyl having carbon atoms of 1 to 4 ; and

$R^3$ is a nitro group in ortho-, meta- or para-position of the phenyl.

2. A process for preparing 1,4-dihydropyridine derivatives represented by the formula (I), which is characterized by reacting a benzaldehyde derivative of the formula (II) with a lower alkyl, lower alkenyl, lower alkoxyalkyl or aminoaryl(lower)alkyl aminocrotonate of the formula(III), and a lower alkyl, lower alkenyl, lower alkoxyalkyl or lower aminoaryl(lower)alkyl acetoacetate of the formula(IV), at a time or in the optional order.

(I)

(II)

(III)

(IV)

wherein
$R^1$ and $R^2$, different from the other, is selected from the group consisting of lower alkyl, lower alkenyl, lower alkoxyalkyl or aminoaryl(lower)alkyl having carbon atoms of 1 to 6, which may be substituted or unsubstituted by a lower alkyl having carbon atoms of 1 to 4, wherein at least one of $R^1$ and $R^2$ is a lower alkenyl having carbon atoms of 3 to 6 whose 1- or 2- position is substituted by a lower alkyl having carbon atoms of 1 to 4 ; and
$R^3$ is a nitro group in ortho-, meta- or para-position of the phenyl.

3. A 1,4-dihydropyridine derivative (e.g. represented by the formula (I)), for use as a medicament for example as a vasodilator or antihypertensive agent.

4. Use of a 1,4-dihydropyridine derivative according to claim 3 for the manufacture of a medicament for vasodilation or for the treatment of hypertension.

5. A pharmaceutical composition having as its active ingredient a 1,4-dihydropyridine derivative according to claim 3.

6. A composition according to claim 5 also comprising an excipient.

7. A process for the manufacture of a 1,4-dihydropyridine derivative according to claim 3 or a composition according to claim 5 or claim 6.

B.P.
H.R.
1min.
Effect Duration Time

FIG. 1

FIG. 2A

●: Systolic Blood Pressure

○: Diastolic Blood Pressure
◉: Heart Rate
20
0
−20
−40
Δ % of Response
1
10
0
100
log Dose μg/kg iv

FIG. 2B

●: Systolic Blood Pressure
○: Diastolic Blood Pressure
◉: Heart Rate

20
0
−20
−40
Δ % of Response
1
3
10
30
100
log Dose μg/kg iv

FIG. 2C

20
0
−20
−40
Δ % of Response
1
3
10
30
100
log Dose μg/kg iv

●: Systolic Blood Pressure
○: Diastolic Blood Pressure
◉: Heart Rate

FIG. 2D

●: Systolic Blood Pressure

○: Diastolic Blood Pressure
⊙: Heart Rate
20
0
−20
−40
Δ% of Response
1
3
10
30
100
log Dose µg/kg iv

FIG. 2E

●: Systolic Blood Pressure
○: Diastolic Blood Pressure
⊙: Heart Rate
20
0
−20
−40
Δ% of Response
1
3
10
30
100
log Dose µg/kg iv

FIG. 2F

20
0
−20
−40
Δ% of Response
1
3
10
30
100
log Dose µg/kg iv

●: Systolic Blood Pressure
○: Diastolic Blood Pressure
⊙: Heart Rate

FIG. 2G

●: Systolic Blood Pressure
○: Diastolic Blood Pressure

⊙: Heart Rate
20
0
−20
−40
Δ% of Response
1
3
10
30
100
log Dose μg/kg iv

FIG. 2H

●: Systolic Blood Pressure
○: Diastolic Blood Pressure
⊙: Heart Rate

20
0
−20
−40
Δ% of Response
1
3
10
30
100
log Dose μg/kg iv

FIG. 2I

20
0
−20
−40
Δ% of Response
1
3
10
30
100
log Dose μg/kg iv

●: Systolic Blood Pressure
○: Diastolic Blood Pressure
⊙: Heart Rate

# FIG. 2J

●: Systolic Blood Pressure
○: Diastolic Blood Pressure
◎: Heart Rate
20
0
−20
−40
Δ% of Response
1
3
10
30
100
log Dose μg/kg iv

# FIG. 2K

●: Systolic Blood Pressure
○: Diastolic Blood Pressure
◎: Heart Rate
20
0
−20
−40
Δ% of Response
1
3
10
30
100
log Dose μg/kg iv

# F I G. 2L

●: Systolic Blood Pressure
○: Diastolic Blood Pressure
⊙: Heart Rate
20
0
−20
−40
Δ% of Response
1
3
10
30
100
log Dose μg/kg iv

FIG. 3

100
50
0
Δ % of Relaxation
$10^{-10}$
$10^{-9}$
$10^{-8}$
$10^{-7}$
$10^{-6}$
Dose (M)
○ : Example 12
◎ : Example 10
● : Example 8

FIG. 4

100
50
0
Δ % of Decrease
$10^{-8}$
$10^{-7}$
$10^{-6}$
$10^{-5}$
$10^{-4}$
Dose (M)
○ : Example 12
◎ : Example 10
● : Example 8

European Patent Office

# EUROPEAN SEARCH REPORT

Application Number

EP 89 30 0280

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| P,X | CHEMICAL ABSTRACTS, vol. 110, 1989, pages 27-28, abstract no. 69116z, Columbus, Ohio, US; Y.K. SHIM et al.: "Synthesis and cardiovascular activities of 1,4-dihydropyridine derivatives", & YAKHAK HOECHI 1988, 32(3), 157-63 * Abstract * | 1,4 | C 07 D 211/90<br>A 61 K 31/445 |
| X | EP-A-0 161 877 (FUJIREBIO INC.) * Claims 1,4 * | 1,4 | |

TECHNICAL FIELDS SEARCHED (Int. Cl.4)

C 07 D 211/00
A 61 K 31/00

The present search report has been drawn up for all claims

| Place of search | Date of completion of the search | Examiner |
|---|---|---|
| THE HAGUE | 06-04-1989 | CASADO Y MARTIN DE MERCA |

CATEGORY OF CITED DOCUMENTS

- X : particularly relevant if taken alone
- Y : particularly relevant if combined with another document of the same category
- A : technological background
- O : non-written disclosure
- P : intermediate document
- T : theory or principle underlying the invention
- E : earlier patent document, but published on, or after the filing date
- D : document cited in the application
- L : document cited for other reasons
- & : member of the same patent family, corresponding document